(19) Europäisches Patentamt European Patent Office Office européen des brevets

(11) **EP 4 321 612 A1**

(12) **EUROPEAN PATENT APPLICATION**
published in accordance with Art. 153(4) EPC

(43) Date of publication:
**14.02.2024 Bulletin 2024/07**

(21) Application number: **21942511.3**

(22) Date of filing: **24.06.2021**

(51) International Patent Classification (IPC):
**C12N 5/0783** (2010.01)   **A61K 35/17** (2015.01)
**A61P 35/00** (2006.01)

(86) International application number:
**PCT/CN2021/102023**

(87) International publication number:
**WO 2022/246942 (01.12.2022 Gazette 2022/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **25.05.2021   CN 202110573668**

(71) Applicant: **Zhaotai Immugene Biomedicine (Hong
Kong) Limited
Hong Kong 999077 (HK)**

(72) Inventors:
• **LI, Peng
Hong Kong 999077 (HK)**

• **LI, Yao
Hong Kong 999077 (HK)**
• **ZHOU, Linfu
Hong Kong 999077 (HK)**
• **TANG, Zhaoyang
Hong Kong 999077 (HK)**
• **QIN, Le
Hong Kong 999077 (HK)**
• **YAO, Yao
Hong Kong 999077 (HK)**
• **HU, Duo
Hong Kong 999077 (HK)**

(74) Representative: **De Bonis, Paolo
Buzzi, Notaro & Antonielli d'Oulx S.p.A.
Corso Vittorio Emanuele II, 6
10123 Torino (IT)**

(54) **INDUCER FOR REPROGRAMMING T CELL INTO NK-LIKE CELL AND APPLICATION OF INDUCER**

(57)    An inducer for reprogramming a T cell into an NK-like cell and an application of the inducer. The inducer comprises any one of or a combination of at least two of a DNA methyltransferase inhibitor, a histone deacetylase inhibitor, or a histone methyltransferase EZH2 inhibitor. The inducer is used for inducing a decrease in methylation level in T cells, inhibiting histone deacetylation and histone methylation, and expressing NK cell receptors and cytokines, thereby achieving the purpose of in-vitro reprogramming of T cells into NK-like cells. The method is simple, high in efficiency, and short in cycle, and the prepared NK-like cells have obvious in-vitro killing effects, and have important significance in the field of cell immunotherapy.

FIG. 15A

**Description**

TECHNICAL FIELD

The present application belongs to the technical field of biomedical and relates to an inducer for reprogramming a T cell into an NK-like cell and an application of the inducer.

BACKGROUND

NK cells, which are an important component of a natural immune system, can kill abnormal cells without activation and have a killing effect not restricted by a major histocompatibility complex (MHC). The NK cells, which are one of the most effective and active immune cells in a human body, can recognize viruses and virus-infected cells through NK-cell receptors (NCRs) such as NKp46 and participate in immune responses of multiple viruses. However, since naturally occurring NK cells in the human body are limited in number and regeneration capability, a cell source becomes a major obstacle to limiting the use of the NK cells in tumor treatment. T-cell receptors (TCRs) on surfaces of T cells recognize virus-infected cells or exogenous antigens and make responses. NK-like cells can not only express NCRs such as NKp46, NKp30, NKp44 and NKG2D but also express TCRs with full functions. Moreover, the NK-like cells have functions of the T cells and the NK cells. The NK-like cells have stronger killing activity and an anti-tumor effect with a broader spectrum than normal NK cells. Moreover, the NK-like cells can proliferate in large numbers under an *in vitro* condition, and NK-like cells proliferating *in vitro* can continue to survive *in vivo* for three weeks or more. Reprogramming the T cells as the NK-like cells provides a brand new cell source for the immunotherapy of tumors and related diseases, thereby significantly promoting the development of tumor cell immunotherapy.

At present, a method for reprogramming T cells is mainly based on a transgenic means, which includes lentiviral transfection, PB system electroporation and CRISPR/cas9 system knockout. However, these methods have many defects, for example, a long treatment period and cumbersome process in the manner of lentiviral transfection, the loss of a large number of primary T cells and relatively low efficiency in the manner of electroporation, an off-target probability in a gene overexpression or knockout process, and the above three manners are all relatively difficult to achieve a large-scale application. Therefore, an effect of large-scale acquisition of NK cells *in vitro* still cannot be achieved fundamentally.

There are no related researches and reports on whether the reprogramming of T cells and the large-scale preparation of NK-like cells can be achieved *in vitro* based on a compound drug in the existing art.

SUMMARY

The present application provides an inducer for reprogramming a T cell into an NK-like cell and an application of the inducer. The inducer is used for treating the T cell so that a demethylation level of the T cell is reduced, processes of the acetylation and methylation of a histone are inhibited, a receptor on a surface of an NK cell is expressed and a cytokine is secreted, thereby achieving an object of reprogramming the T cell into the NK-like cell *in vitro.*

In a first aspect, the present application provides an inducer for reprogramming a T cell into an NK-like cell. The inducer includes any one or a combination of at least two of a DNA methyltransferase inhibitor, a histone deacetylase inhibitor or a histone methyltransferase EZH2 inhibitor.

In the present application, a small molecule drug DNA methyltransferase inhibitor is used for treating the T cell so that the T cell expresses an NK-cell receptor, thereby achieving an object of reprogramming the T cell into the NK-like cell *in vitro.*

Preferably, the DNA methyltransferase inhibitor includes a DNA methyltransferase 1 inhibitor, preferably decitabine and/or GSK-3484862.

In the present application, DNA methyltransferase 1 (DNMT1) is the most important methyltransferase in a human body and also has an ability to regulate cell cycle and regulate the expression of a tumor suppressor gene. Decitabine (DAC), which is a cytosine analogue and belongs to a specific methyltransferase inhibitor, can covalently bind to a DNA methyltransferase to inhibit the activity of the enzyme so that DNA is demethylated and a tumor suppressor gene is re-expressed. GSK-3484862 is another non-covalent inhibitor of the DNMT1 that achieves an anti-cancer effect by inducing DNA hypomethylation.

In the present application, using decitabine and/or GSK-3484862 for inducing the reduction of a methylation level of the T cell and expressing an NK cell to kill a related molecule achieves the object of reprogramming the T cell into the NK-like cell *in vitro.*

Preferably, the histone deacetylase inhibitor includes any one or a combination of at least two of Mocetinostat, Givinostat or Entinostat.

A histone deacetylase (HDAC) plays an important role in the structural modification of a chromosome and the regulation of gene expression. The HDAC can inhibit the relaxation of a nucleosome, thereby inhibiting the binding of various transcription factors and synergistic transcription factors to DNA sites. The HDAC can interact with other chromatin regulators to regulate an epigenetic process. Mocetinostat, Givinostat (ITF2357) and Entinostat (MS-275) are all new heteroselective inhibitors of the HDAC.

Preferably, the histone methyltransferase inhibitor includes Tazemetostat and/or GSK126.

An enhancer of zeste homolog 2 (EZH2) of a histone methyltransferase, which is a catalytic subunit of an enzyme of a polycomb repressive complex 2 (PRC2), can perform methylation modification on a 27th lysine (H3K27) in a histone H3 of the nucleosome, resulting in the silencing of a downstream target gene. Therefore, the enhancer plays an important role in important biological processes such as cell apoptosis, cell cycle and cell differentiation. Tazemetostat is an oral EZH2 small molecule inhibitor, and GSK126 (GSK2816126) is a new selective enzyme activity inhibitor.

Preferably, the inducer further includes a pharmaceutically acceptable adjuvant.

Preferably, the adjuvant includes any one or a combination of at least two of a carrier, a diluent, an excipient, a filler, an adhesive, a wetting agent, a disintegrant, an emulsifier, a co-solvent, a solubilizer, a osmotic pressure adjuster, a surfactant, a coating material, a colorant, a pH adjusting agent, an anti-oxidant, an bacteriostatic agent or a buffering agent.

In a second aspect, the present application provides a method for reprogramming a T cell into an NK-like cell, and the method includes:

co-culturing an activated T cell with the inducer according to the first aspect to obtain the NK-like cell.

Preferably, the inducer includes any one or a combination of at least two of decitabine, GSK-3484862, Mocetinostat, Givinostat, Entinostat, Tazemetostat or GSK126.

Preferably, decitabine has a final concentration of 0.05-0.5 $\mu$M, which may be, for example, 0.05 $\mu$M, 0.1 $\mu$M, 0.2 $\mu$M, 0.3 $\mu$M, 0.4 $\mu$M or 0.5 $\mu$M.

Preferably, GSK-3484862 has a final concentration of 0.5-8 $\mu$M, which may be, for example, 0.5 $\mu$M, 1 $\mu$M, 2 $\mu$M, 3 $\mu$M, 4 $\mu$M, 5 $\mu$M, 6 $\mu$M, 7 $\mu$M or 8 $\mu$M.

Preferably, Mocetinostat has a final concentration of 0.1-0.5 $\mu$M, which may be, for example, 0.1 $\mu$M, 0.2 $\mu$M, 0.3 $\mu$M, 0.4 $\mu$M or 0.5 $\mu$M.

Preferably, Givinostat has a final concentration of 0.05-1 $\mu$M, which may be, for example, 0.05 $\mu$M, 0.1 $\mu$M, 0.2 $\mu$M, 0.3 $\mu$M, 0.4 $\mu$M, 0.5 $\mu$M, 0.6 $\mu$M, 0.7 $\mu$M, 0.8 $\mu$M, 0.9 $\mu$M or 1 $\mu$M.

Preferably, Entinostat has a final concentration of 0.05-1 $\mu$M, which may be, for example, 0.05 $\mu$M, 0.1 $\mu$M, 0.2 $\mu$M, 0.3 $\mu$M, 0.4 $\mu$M, 0.5 $\mu$M, 0.6 $\mu$M, 0.7 $\mu$M, 0.8 $\mu$M, 0.9 $\mu$M or 1 $\mu$M.

Preferably, Tazemetostat has a final concentration of 0.1-5 $\mu$M, which may be, for example, 0.1 $\mu$M, 0.5 $\mu$M, 1 $\mu$M, 2 $\mu$M, 3 $\mu$M, 4 $\mu$M or 5 $\mu$M.

Preferably, GSK126 has a final concentration of 0.05-1 $\mu$M, which may be, for example, 0.05 $\mu$M, 0.1 $\mu$M, 0.2 $\mu$M, 0.3 $\mu$M, 0.4 $\mu$M, 0.5 $\mu$M, 0.6 $\mu$M, 0.7 $\mu$M, 0.8 $\mu$M, 0.9 $\mu$M or 1 $\mu$M.

Preferably, the co-culture is performed for 3-10 days, which may be, for example, 3 days, 4 days, 5 days, 6 days, 7 days, 8 days, 9 days or 10 days, preferably 5 days.

Preferably, the method for reprogramming a T cell into an NK-like cell includes:

adding any one or a combination of at least two of decitabine having the final concentration of 0.05-0.5 $\mu$M, GSK-3484862 having the final concentration of 0.5-8 $\mu$M, GSK126 having the final concentration of 0.05-1 $\mu$M, Mocetinostat having the final concentration of 0.1-0.5 $\mu$M, Givinostat having the final concentration of 0.05-1 $\mu$M, Entinostat having the final concentration of 0.05-1 $\mu$M or Tazemetostat having the final concentration of 0.1-5 $\mu$M to the activated T cell, culturing for 3-10 days, and changing half of a medium every day to obtain the NK-like cell.

In a third aspect, the present application provides an NK-like cell prepared through the method according to the second aspect. The NK-like cell expresses an NK-cell receptor and a T-cell receptor.

Preferably, the NK-cell receptor includes any one or a combination of at least two of NKp46, NKp30, NKp44 or NKG2D.

In a fourth aspect, the present application provides a pharmaceutical composition. The pharmaceutical composition includes the NK-like cell according to the third aspect.

Preferably, the pharmaceutical composition further includes any one or a combination of at least two of a pharmaceutically acceptable carrier, excipient or diluent.

In a fifth aspect, the present application provides use of the inducer according to the first aspect, the NK-like cell according to the third aspect or the pharmaceutical composition according to the fourth aspect for preparing a cell immunotherapy drug.

Compared with the prior art, the present application has the beneficial effects described below.

(1) In the present application, any one or a combination of at least two of the small molecule drug DNA methyltransferase inhibitor, the histone deacetylase inhibitor or the histone methyltransferase EZH2 inhibitor is used for treating the T cell so that the T cell expresses the NK-cell receptor, thereby achieving the object of reprogramming the T cell into the NK-like cell *in vitro.*

(2) In the present application, the high expression of NK-cell receptors NKp46 and NKp30 is significant in the reprogrammed T cell induced by the above inducer, and the reprogrammed T cell has an apparent *in vitro* killing effect and stable dual functions of the T cell and the NK cell; the reprogrammed T cell can also secrete cytokines such as IFN-γ and a granzyme B for an immune effect.

(3) The method for preparing an NK-like cell of the present application with a simple process and high reprogramming efficiency significantly shortens an *in vitro* reprogramming period of the T cell and improves a utilization rate of a primary T cell. Therefore, the method is suitable for large-scale process popularization and of great significance in the field of cell immunotherapy.

BRIEF DESCRIPTION OF DRAWINGS

FIG. 1A is a diagram illustrating the expression of NKp30 and NKp46 in CD4 T cells among T cells treated by DMSO, and FIG. 1B is a diagram illustrating the expression of NKp30 and NKp46 in CD4 T cells among T cells induced by DAC.

FIG. 2A is a diagram illustrating the expression of NKp30 and NKp46 in CD8 T cells among the T cells treated by DMSO, and FIG. 2B is a diagram illustrating the expression of NKp30 and NKp46 in CD8 T cells among the T cells induced by DAC.

FIG. 3A is the diagram illustrating the expression of NKp30 and NKp46 in the CD4 T cells among the T cells treated by DMSO, and FIG. 3B is a diagram illustrating the expression of NKp30 and NKp46 in CD4 T cells among T cells induced by GSK-3484862.

FIG. 4A is the diagram illustrating the expression of NKp30 and NKp46 in the CD8 T cells among the T cells treated by DMSO, and FIG. 4B is a diagram illustrating the expression of NKp30 and NKp46 in CD8 T cells among the T cells induced by GSK-3484862.

FIG. 5A is a diagram illustrating the expression of NKp30 and NKp46 in the CD4 T cells among the T cells treated by DMSO, and FIG. 5B is a diagram illustrating the expression of NKp30 and NKp46 in CD4 T cells among T cells co-induced by DAC and Mocetinostat.

FIG. 6A is a diagram illustrating the expression of NKp30 and NKp46 in the CD8 T cells among the T cells treated by DMSO, and FIG. 6B is a diagram illustrating the expression of NKp30 and NKp46 in CD8 T cells among the T cells co-induced by DAC and Mocetinostat.

FIG. 7A is the diagram illustrating the expression of NKp30 and NKp46 in the CD4 T cells among the T cells treated by DMSO, and FIG. 7B is a diagram illustrating the expression of NKp30 and NKp46 in CD4 T cells among T cells co-induced by GSK-3484862 and Mocetinostat.

FIG. 8A is a diagram illustrating the expression of NKp30 and NKp46 in the CD8 T cells among the T cells treated by DMSO, and FIG. 8B is a diagram illustrating the expression of NKp30 and NKp46 in CD8 T cells among the T cells co-induced by GSK-3484862 and Mocetinostat.

FIG. 9A is a diagram illustrating the expression of NKp30 and NKp46 in the CD4 T cells among the T cells treated by DMSO, and FIG. 9B is a diagram illustrating the expression of NKp30 and NKp46 in the CD4 T cells among the T cells induced by GSK-3484862.

FIG. 10A is a diagram illustrating the expression of NKp30 and NKp46 in the CD8 T cells among the T cells treated by DMSO, and FIG. 10B is a diagram illustrating the expression of NKp30 and NKp46 in the CD8 T cells among the T cells induced by GSK-3484862.

FIG. 11A is a diagram illustrating the expression of NKp30 and NKp46 in CD4 T cells among T cells treated by Tazemetostat, and FIG. 11B is a diagram illustrating the expression of NKp30 and NKp46 in CD4 T cells among T cells induced by GSK126.

FIG. 12A is a diagram illustrating the expression of NKp30 and NKp46 in CD8 T cells among the T cells treated by Tazemetostat, and FIG. 12B is a diagram illustrating the expression of NKp30 and NKp46 in CD8 T cells among the T cells induced by GSK126.

FIG. 13A is a diagram illustrating the expression of NKp30 and NKp46 in CD4 T cells among T cells co-induced by Tazemetostat and GSK-3484862, and FIG. 13B is a diagram illustrating the expression of NKp30 and NKp46 in CD4 T cells among T cells co-induced by GSK126 and GSK-3484862.

FIG. 14A is a diagram illustrating the expression of NKp30 and NKp46 in CD8 T cells among the T cells co-induced by Tazemetostat and GSK-3484862, and FIG. 14B is a diagram illustrating the expression of NKp30 and NKp46 in CD8 T cells among the T cells co-induced by GSK126 and GSK-3484862.

FIG. 15A is a diagram illustrating *in vitro* killing results of the T cells induced by GSK-3484862, the T cells induced by DAC, the T cells induced by Mocetinostat, the T cells co-induced by GSK-3484862 and Mocetinostat, the T cells co-induced by DAC and Mocetinostat and the T cells treated by DMSO against K562.

FIG. 15B is a diagram illustrating *in vitro* killing results of the T cells induced by GSK-3484862, the T cells induced by Tazemetostat, the T cells induced by GSK126, the T cells co-induced by GSK-3484862 and Tazemetostat, the T cells co-induced by GSK-3484862 and GSK126 and the T cells treated by DMSO against K562.

FIG. 16A is a diagram illustrating a result of a level of IFN-$\gamma$ secreted after the T cells induced by DAC are co-cultured with K562, and FIG. 16B is a diagram illustrating a result of a level of a granzyme B secreted after the T cells induced by DAC are co-cultured with K562.

FIG. 17A is a diagram illustrating a result of a level of IFN-$\gamma$ secreted after the T cells induced by GSK-3484862 are co-cultured with K562, and FIG. 17B is a diagram illustrating a result of a level of a granzyme B secreted after the T cells induced by GSK-3484862 are co-cultured with K562.

FIG. 18A is a diagram illustrating a result of a level of IFN-$\gamma$ secreted after the T cells co-induced by DAC and Mocetinostat are co-cultured with K562, and FIG. 18B is a diagram illustrating a result of a level of a granzyme B secreted after the T cells co-induced by DAC and Mocetinostat are co-cultured with K562.

FIG. 19A is a diagram illustrating a result of a level of IFN-$\gamma$ secreted after the T cells co-induced by GSK-3484862 and Mocetinostat are co-cultured with K562, and FIG. 19B is a diagram illustrating a result of a level of a granzyme B secreted after the T cells co-induced by GSK-3484862 and Mocetinostat are co-cultured with K562.

DETAILED DESCRIPTION

To further elaborate on the technical means adopted and effects achieved in the present application, the present application is further described below in conjunction with examples and drawings. It is to be understood that the specific examples set forth below are intended to explain the present application and not to limit the present application.
Experiments without specific techniques or conditions specified in the examples are conducted according to techniques or conditions described in the literature in the art or product specifications. The reagents or instruments used herein without manufacturers specified are conventional products commercially available from proper channels.

Materials:

umbilical cord blood from Guangdong Cord Blood Bank;

Pan T cell isolation kit available from STEMCELL Technologies (Canada);

TransAct available from Miltenyi Biotec in Germany;

decitabine available from Selleck (Shanghai Blue-Wood Chemicals Co., Ltd., China);

GSK-3484862, GSK126, Mocetinostat, Givinostat, Entinostat and Tazemetostat available from MCE (MedChemExpress);

CD3 PE-Cy7, CD4 APC-Cy7, CD8 FITC, NKp30 PE and NKp46 APC available from BioLegend (America);

K562 cells from ATCC;

ELISA assay reagents available from Dakewe Biotech Co., Ltd.

**Example 1 Isolation and *in vitro* culture of primary T cells in human umbilical cord blood**

In this example, umbilical cord blood mononuclear cells (UCBMCs) were separated from the umbilical cord blood through Ficoll-hypaque (polysucrose-meglumine diatrizoate) density gradient centrifugation, $4 \times 10^7$ CD3 positive UCBMCs were taken and cultured overnight with a cell density of $2 \times 10^6$ cells/mL, remaining UCBMCs were frozen, and T cells were sorted by the Pan T cell isolation kit and cultured for a period of time to obtain $\geq 4 \times 10^7$ CD3 positive T cells with a viability rate of $\geq 70\%$ and without contamination by exogenous microorganisms such as bacteria, fungi and mycoplasmas.

**Example 2 T cell reprogramming induced by small molecule drugs**

(1) T cells activated by TransAct for 36 h were taken and centrifuged at 300 g for 5 min, the T cells were resuspended with an IMDM+5%FBS+1% double antibody (a $100 \times$ penicillin-streptomycin mixed solution)+IL2 (300 U), and a T cell density was adjusted to $(2-3) \times 10^5$ cells/mL.
(2) Groups of small molecule inhibitor drugs were added to the resuspended T cells, respectively. Final concentrations of the drugs added are shown in Table 1. Half of media were changed every day, and extra new drugs were added according to volumes obtained after the media were changed. A day when the drugs were initially added was denoted as Day0, and when the drugs continued to be added until Day5, the mixtures were centrifuged at 300 g for 5 min, and the media were changed. Then, the cells continued to be cultured with an IMDM+5%FBS+1% double antibody (a $100 \times$ penicillin-streptomycin mixed solution).

Table 1

| Group | Drug (Concentration) |
|---|---|
| 1 | DAC (0.2 $\mu$M) |
| 2 | GSK-3484862 (2 $\mu$M) |
| 3 | Mocetinostat (0.2 $\mu$M) |
| 4 | Tazemetostat (2.5 $\mu$M) |
| 5 | GSK126 (0.1 $\mu$M) |
| 6 | DAC+Mocetinostat (0.2 $\mu$M+0.2 $\mu$M) |
| 7 | GSK-3484862+Mocetinostat (2 $\mu$M+0.2 $\mu$M) |
| 8 | Tazemetostat+GSK3484862 (2.5 $\mu$M+1 $\mu$M) |
| 9 | GSK126+GSK-3484862 (0.1 $\mu$M+1 $\mu$M) |

**Example 3 Detection of phenotypes of reprogrammed T cells through flow cytometry**

(1) 200 $\mu$L each of the T cells induced by DAC until Day6, the T cells induced by GSK-3484862 until Day 6, the T cells co-induced by DAC and Mocetinostat until Day6, the T cells co-induced by GSK-3484862 and Mocetinostat until Day6 and the untreated T cells were taken and centrifuged at 400 g for 4 min. After supernatant was discarded, 50 $\mu$L phosphate buffer (PBS) was added to resuspend the cells. Then, 0.5 $\mu$L each of CD3 PE-Cy7, CD4 APC-Cy7, CD8 FITC, NKp30 PE and NKp46 APC were added to each of the mixtures and incubated for 30 min at 4°C in the dark, 500 $\mu$L PBS was added to dilute the antibody and centrifuged at 400 g for 4 min, supernatant was carefully discarded, and 300 $\mu$L PBS was added to resuspend the cells, transferred to a flow tube and loaded on a flow cytometer.

(2) Data analysis was performed by BD-flowcyto analysis software, and 10000 cells were collected in each tube. The appearance of a significant increase in NKp-30 and the appearance of an NKp46-positive cell population were used as signs of successful reprogramming, and a percentage of positive T cells was calculated.

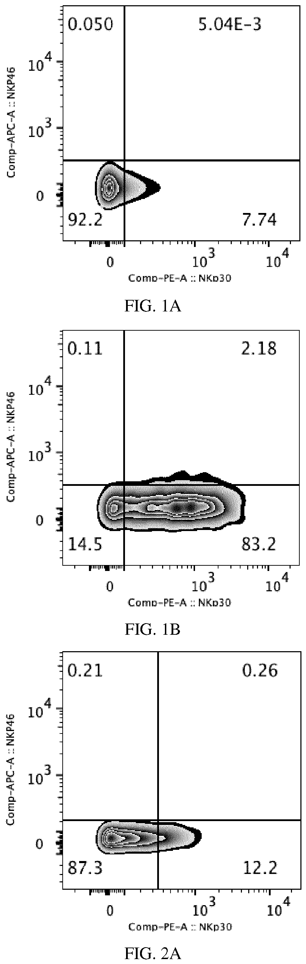

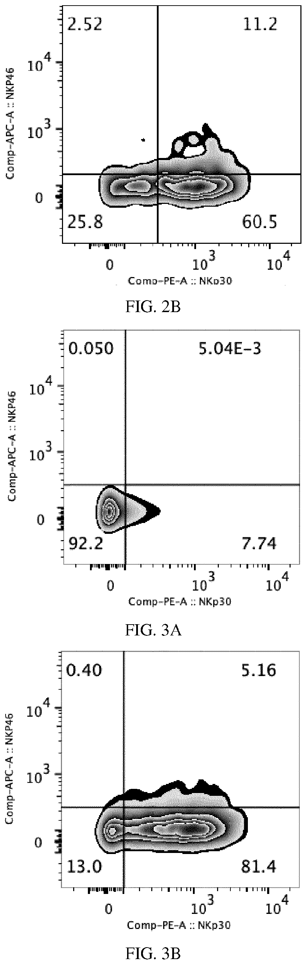

As shown in FIGS. 1A, 1B, 2A and 2B, an expression rate of NKp30 in CD4 T cells in the DAC induction group is approximately 83.2%, while an expression rate of NKp30 in CD4 T cells in the DMSO group is approximately 7.74%; in CD8 T cells in the decitabine induction group, an expression rate of NKp46 is approximately 11.2%, and an expression rate of NKp30 is approximately 60.5%, while in CD8 T cells in the DMSO group, an expression rate of NKp46 is approximately 0.26%, and an expression rate of NKp30 is approximately 12.2%. It indicates that decitabine can induce the T cells to express NKp30 and NKp46.

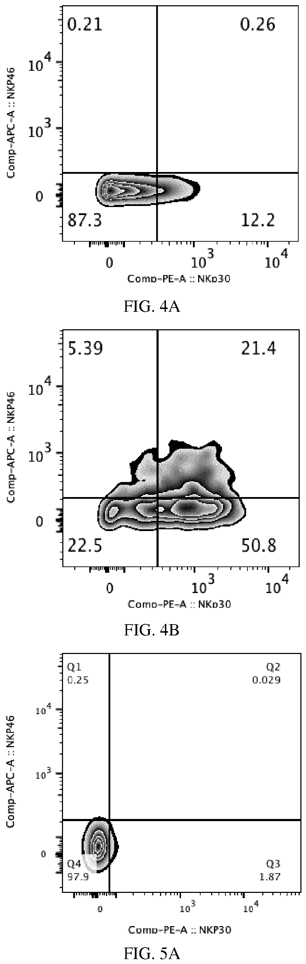

As shown in FIGS. 3A, 3B, 4A and 4B, in CD4 T cells in the GSK-3484862 induction group, an expression rate of NKp46 is approximately 5.16%, and an expression rate of NKp30 is approximately 81.4%, while the expression rate of NKp30 in the CD4 T cells in the DMSO group is approximately 7.74%; in CD8 T cells in the GSK-3484862 induction group, an expression rate of NKp46 is approximately 21.4%, and an expression rate of NKp30 is approximately 50.8%, while in the CD8 T cells in the DMSO group, the expression rate of NKp46 is approximately 0.26%, and the expression rate of NKp30 is approximately 12.2%. It indicates that GSK-3484862 can induce the T cells to express NKp30 and NKp46.

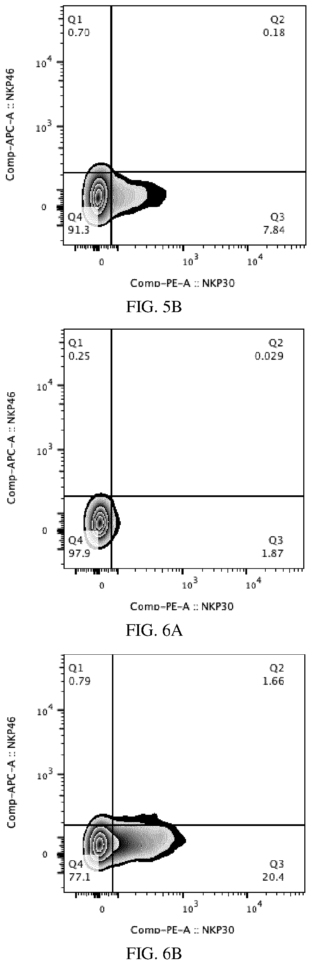

As shown in FIGS. 5A, 5B, 6A and 6B, compared with the T cells induced by DMSO, in CD4 T cells among the T cells co-induced by DAC and Mocetinostat, an expression amount of NKp30 is 7.84%, while NKp46 is hardly expressed; in addition, CD8 T cells among the T cells co-induced by DAC and Mocetinostat, an expression amount of NKp30 is 20.4%, while NKp46 is also hardly expressed.

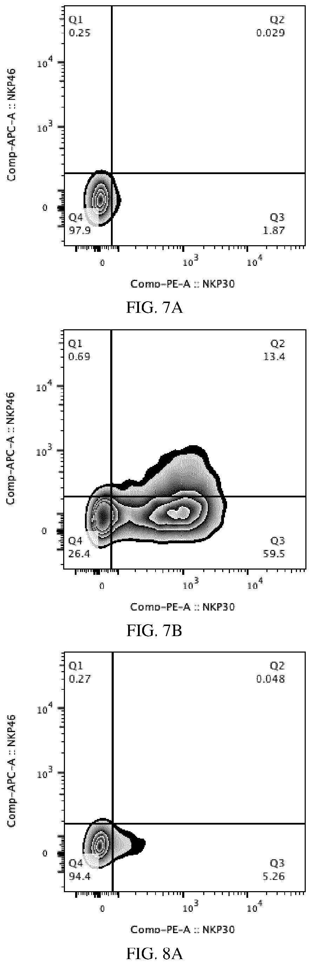

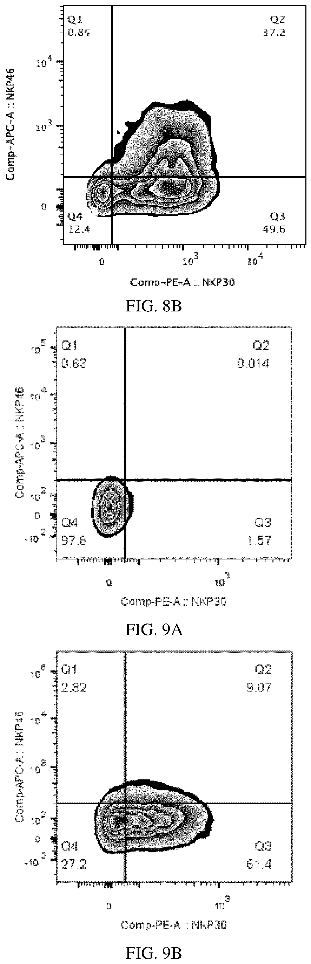

As shown in FIGS. 7A, 7B, 8A and 8B, among the T cells co-induced by GSK-3484862 and Mocetinostat, in CD4 T cells, an expression amount of NKp30 is 59.5%, and an expression amount of NKp46 is 13.4%, while in CD8 cells, an expression amount of NKp30 is 49.6%, and an expression amount of NKp46 is 37.2%, indicating that compared with the results of the co-induction by DAC and Mocetinostat, expression levels of NKp30 and NKp46 in the T cells induced by GSK-3484862 and Mocetinostat are both significantly improved.

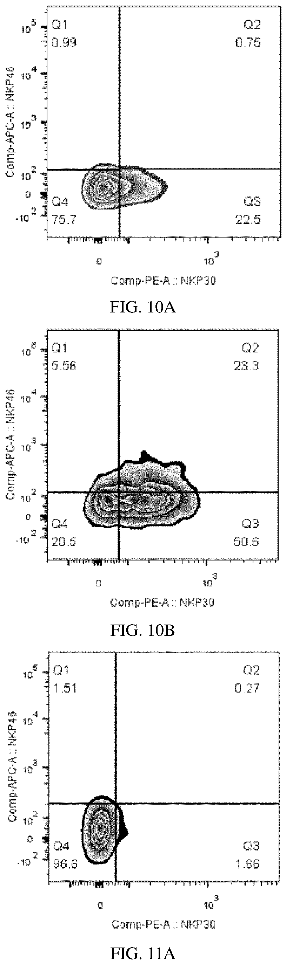

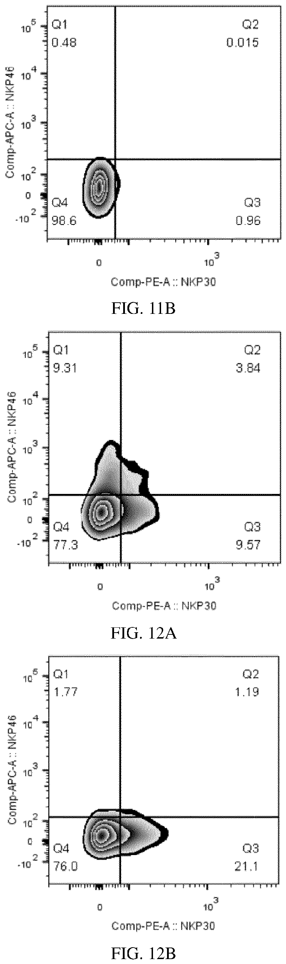

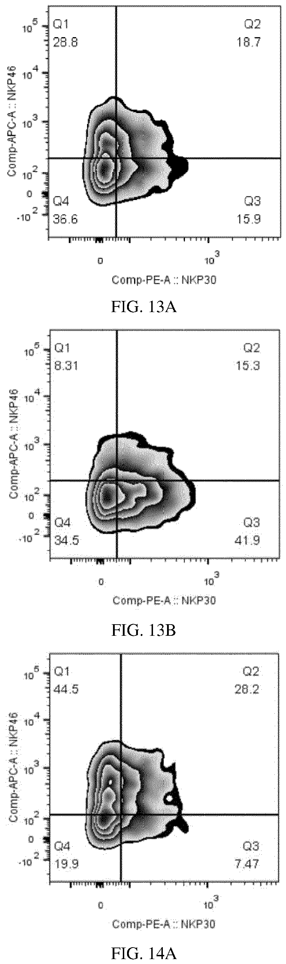

In addition, the T cells induced by GSK-3484862 until Day5, the T cells induced by Tazemetostat until Day5, the T cells induced by GSK126 until Day5, the T cells co-induced by Tazemetostat and GSK-3484862 until Day5, the T cells co-induced by GSK-3484862 and GSK126 until Day5 and the untreated T cells were taken and subjected to the same operations, and phenotypes of the reprogrammed T cells were identified through flow cytometry. The results are shown in FIGS. 9A, 9B, 10A, 10B, 11A, 11B, 12A, 12B, 13A, 13B, 14A and 14B. As can be seen from the figures, compared with DMSO, both NKp46 and NKp30 are significantly expressed in CD4 and CD8 among T cells induced by a single inhibitor; further, after GSK-3484862 is combined with Tazemetostat and GSK126, expression amounts of NKp46 in CD4 and CD8 among the induced T cells are both significantly improved. The above results indicate that either the single inhibitor (Tazemetostat, GSK126 or GSK-3484862) or the combination of double inhibitors (Tazemetostat+GSK-3484862 or GSK-3484862+GSK126) can induce the T cells to reprogram into NK-like cells.

**Example 4 *In vitro* killing detection of reprogrammed T cells induced by small molecule drugs**

(1) The T cells induced by GSK-3484862, the T cells induced by DAC, the T cells induced by Mocetinostat, the T cells co-induced by GSK-3484862 and Mocetinostat, the T cells co-induced by DAC and Mocetinostat and the T cells treated by DMSO were mixed with $1 \times 10^4$ K562 cells (human chronic myeloid leukemia cells) according to different effector-target ratios (16:1, 8:1, 4:1, 2:1, 1:1, 1:2 and 1:4), respectively, and added to a 96-well cell culture plate. Three duplicate wells were set for each group, and a group with the addition of tumor cells alone was set as a positive control. After centrifugation at $250 \times g$ for 5 min, the cells were placed in a 5% $CO_2$ incubator at 37°C and co-cultured for 24 h.

(2) After 24 h, 100 μL/well luciferase substrate ($1\times$) was added to the 96-well cell culture plate, and the cells were resuspended and uniformly mixed. RLU (Relative light units) were immediately measured by a multifunctional microplate reader for 0.1 s. A killing proportion calculation formula of a quantitative killing efficiency assessment method using a luciferase is as follows:

100%×(control well reading-experimental well reading)/control well reading (blank group (without the addition of the cells) reading may be ignored).

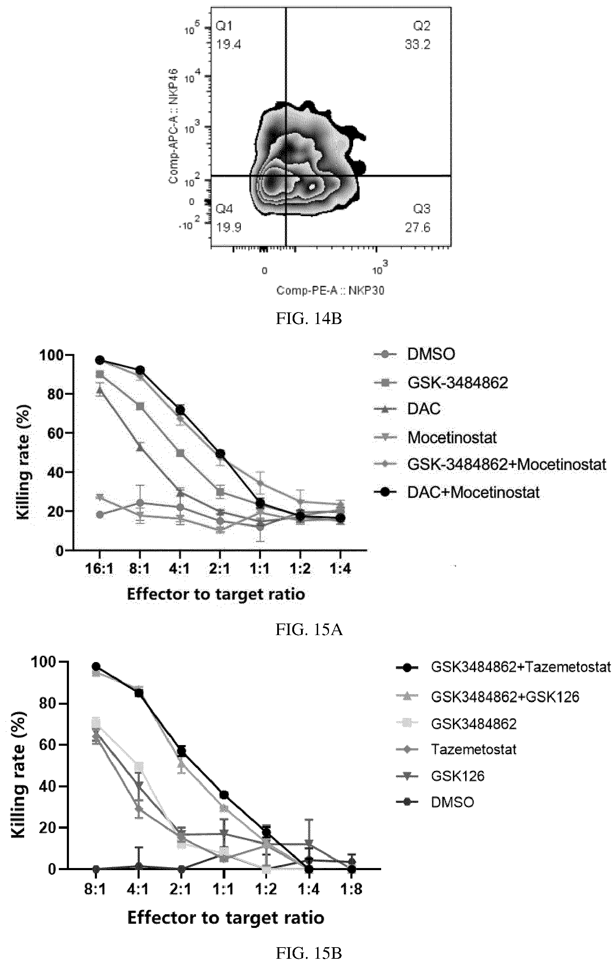

The results are shown in FIG. 15A. The T cells induced by GSK-3484862, the T cells induced by DAC, the T cells induced by Mocetinostat, the T cells co-induced by GSK-3484862 and Mocetinostat and the T cells co-induced by DAC and Mocetinostat can all effectively kill the human chronic myeloid leukemia cells.

In addition, according to the same method, the T cells induced by GSK-3484862, the T cells induced by Tazemetostat, the T cells induced by GSK126, the T cells co-induced by GSK-3484862 and Tazemetostat and the T cells co-induced by GSK-3484862 and GSK-126 were separately used and the T cells treated by DMSO was used as a control to detect killing abilities of the reprogrammed T cells against K562 cells. The results are shown in FIG. 15B.

The results indicate that the T cells induced by GSK-3484862, the T cells induced by Tazemetostat, the T cells induced by GSK126, the T cells co-induced by GSK-3484862 and Tazemetostat and the T cells co-induced by GSK-3484862 and GSK126 can all effectively kill the human chronic myeloid leukemia cells.

**Example 5 Secretion of cytokines by reprogrammed T cells induced by small molecule drugs**

The T cells induced by DAC, the T cells induced by GSK-3484862, the T cells co-induced by DAC and Mocetinostat, the T cells co-induced by GSK-3484862 and Mocetinostat and the T cells treated by DMSO were co-cultured with human chronic myeloid leukemia cells K562 for 48 h, respectively, and expression levels of cytokines IFN-$\gamma$ and a granzyme B in supernatant were detected through ELISA.

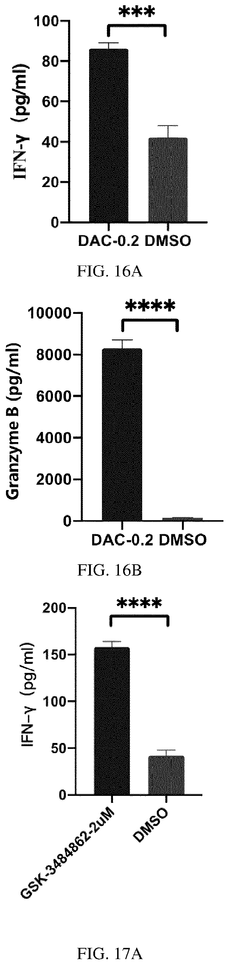

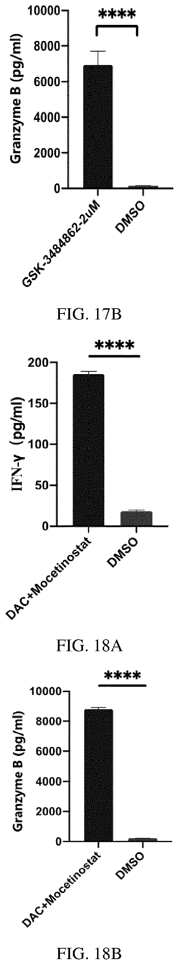

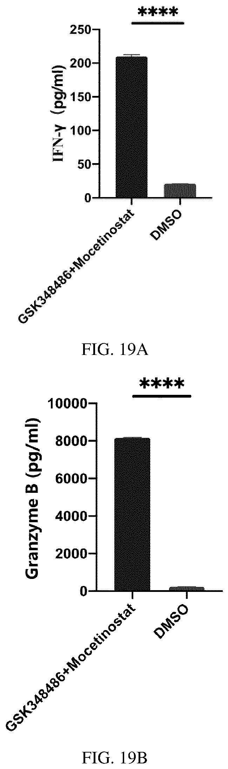

The results are shown in FIGS. 16A, 16B, 17A, 17B, 18A, 18B, 19A and 19B, indicating that the T cells induced by DAC, the T cells induced by GSK-3484862, the T cells co-induced by DAC and Mocetinostat and the T cells co-induced by GSK-3484862 and Mocetinostat can all produce an immune effect.

To conclude, in the present application, decitabine and/or GSK-3484862 are used for inducing the T cells into NK-like cells with a simple method which is high in efficiency, short in period and suitable for large-scale preparation, and the obtained NK-like cells have an apparent *in vitro* killing effect, stable dual functions of the T cells and the NK cells and an important application prospect in the field of cell immunotherapy.

The applicant has stated that although the detailed method of the present application is described through the examples described above, the present application is not limited to the detailed method described above, which means that the implementation of the present application does not necessarily depend on the detailed method described above. It should be apparent to those skilled in the art that any improvements made to the present application, equivalent replacements of raw materials of the product of the present application, additions of adjuvant ingredients, selections of specific manners, etc., all fall within the protection scope and the disclosure scope of the present application.

**Claims**

1. An inducer for reprogramming a T cell into an NK-like cell, comprising any one or a combination of at least two of a DNA methyltransferase inhibitor, a histone deacetylase inhibitor or a histone methyltransferase EZH2 inhibitor.

2. The inducer according to claim 1, wherein the DNA methyltransferase inhibitor comprises a DNA methyltransferase 1 inhibitor, preferably decitabine and/or GSK-3484862.

3. The inducer according to claim 1 or 2, wherein the histone deacetylase inhibitor comprises any one or a combination of at least two of Mocetinostat, Givinostat or Entinostat.

4. The inducer according to any one of claims 1 to 3, wherein the histone methyltransferase inhibitor comprises Tazemetostat and/or GSK126.

5. The inducer according to any one of claims 1 to 4, wherein the inducer further comprises a pharmaceutically acceptable adjuvant.

6. The inducer according to claim 5, wherein the adjuvant comprises any one or a combination of at least two of a carrier, a diluent, an excipient, a filler, an adhesive, a wetting agent, a disintegrant, an emulsifier, a co-solvent, a solubilizer, a osmotic pressure adjuster, a surfactant, a coating material, a colorant, a pH adjusting agent, an antioxidant, an bacteriostatic agent or a buffering agent.

7. A method for reprogramming a T cell into an NK-like cell, comprising:
co-culturing an activated T cell with the inducer according to any one of claims 1 to 6 to obtain the NK-like cell.

8. The method according to claim 7, wherein the inducer comprises any one or a combination of at least two of decitabine, GSK-3484862, Mocetinostat, Givinostat, Entinostat, Tazemetostat or GSK126;

> preferably, decitabine has a final concentration of 0.05-0.5 $\mu$M;
> preferably, GSK-3484862 has a final concentration of 0.5-8 $\mu$M;
> preferably, Mocetinostat has a final concentration of 0.1-0.5 $\mu$M;
> preferably, Givinostat has a final concentration of 0.05-1 $\mu$M;
> preferably, Entinostat has a final concentration of 0.05-1 $\mu$M;
> preferably, Tazemetostat has a final concentration of 0.1-5 $\mu$M;
> preferably, GSK126 has a final concentration of 0.05-1 $\mu$M.

9. The method according to claim 7 or 8, wherein the co-culture is performed for 3-10 days.

10. The method according to any one of claims 7 to 9, wherein the method comprises:
adding any one or a combination of at least two of decitabine having the final concentration of 0.05-0.5 $\mu$M, GSK-3484862 having the final concentration of 0.5-8 $\mu$M, GSK126 having the final concentration of 0.05-1 $\mu$M, Mocetinostat having the final concentration of 0.1-0.5 $\mu$M, Givinostat having the final concentration of 0.05-1 $\mu$M, Entinostat having the final concentration of 0.05-1 $\mu$M or Tazemetostat having the final concentration of 0.1-5 $\mu$M to the activated T cell, culturing for 3-10 days, and changing half of a medium every day to obtain the NK-like cell.

11. An NK-like cell prepared through the method according to any one of claims 7 to 10, wherein the NK-like cell expresses an NK-cell receptor and a T-cell receptor;
preferably, the NK-cell receptor comprises any one or a combination of at least two of NKp46, NKp30, NKp44 or NKG2D.

12. A pharmaceutical composition, comprising the NK-like cell according to claim 11;
preferably, the pharmaceutical composition further comprises any one or a combination of at least two of a pharmaceutically acceptable carrier, excipient or diluent.

13. Use of the inducer according to any one of claims 1 to 6, the NK-like cell according to claim 11 or the pharmaceutical composition according to claim 12 for preparing a cell immunotherapy drug.

FIG. 1A

FIG. 1B

FIG. 2A

FIG. 2B

FIG. 3A

FIG. 3B

FIG. 4A

FIG. 4B

FIG. 5A

FIG. 5B

FIG. 6A

FIG. 6B

FIG. 7A

FIG. 7B

FIG. 8A

FIG. 8B

FIG. 9A

FIG. 9B

FIG. 10A

FIG. 10B

FIG. 11A

FIG. 11B

FIG. 12A

FIG. 12B

FIG. 13A

FIG. 13B

FIG. 14A

FIG. 14B

FIG. 15A

FIG. 15B

19

FIG. 16A

FIG. 16B

FIG. 17A

FIG. 17B

FIG. 18A

FIG. 18B

FIG. 19A

FIG. 19B

## INTERNATIONAL SEARCH REPORT

| International application No. |
|---|
| **PCT/CN2021/102023** |

**A.    CLASSIFICATION OF SUBJECT MATTER**

C12N 5/0783(2010.01)i;   A61K 35/17(2015.01)i;   A61P 35/00(2006.01)i

According to International Patent Classification (IPC) or to both national classification and IPC

**B.    FIELDS SEARCHED**

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

CNABS, CNTXT, SIPOABS, DWPI, WOTXT, EPTXT, USTXT, CNKI, 万方, WANFANG, 读秀, DUXIU, PUBMED, ISI_Web of Science, Science Direct, STNext: 昭泰英基, 李鹏, 结构检索, structure search, DNA甲基转移酶抑制剂, 组蛋白去乙酰酶抑制剂, 组蛋白甲基转移酶EZH2抑制剂, 地西他滨, GSK-3484862, mocetinostat, givinostat, entinostat, tazemetostat, gsk126, DNMT, HDAC

**C.    DOCUMENTS CONSIDERED TO BE RELEVANT**

| Category* | Citation of document, with indication, where appropriate, of the relevant passages | Relevant to claim No. |
|---|---|---|
| X | 牛超, (NIU, Chao). "地西他滨对γδT细胞功能的影响及机制研究, ( The Roles of Decitabine on the Function of γδT Cells and the Underlying Mechanisms, )" *中国博士学位论文全文数据库 (Chinese Doctoral Dissertations Full-Text Database)*, No. 12, 15 December 2018 (2018-12-15), pp. 1-101 | 1-13 |
| X | OTSUKA, Y. et al.,. "EZH2 inhibitors restore epigenetically silenced CD58 expression in B-cell lymphomas," *Molecular Immunology*, Vol. (119), 18 January 2020 (2020-01-18), pp. 35-45 | 1-13 |
| X | CN 109563043 A (GLAXOSMITHKLINE IP DEV LTD. et al.) 02 April 2019 (2019-04-02) embodiment 3, and claims 1-6 and 40 | 1-6, 13 |
| X | CN 109715173 A (VIRACTA THERAPEUTICS INC.) 03 May 2019 (2019-05-03) claims 1 and 2 | 1-6, 13 |
| X | WO 2020165402 A1 (WESTFÄLISCHE WILHELMS-UNIVERSITÄT MÜNSTER) 20 August 2020 (2020-08-20) abstract, claims 1-20, and description, pages 9-10, table 1 | 1-13 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **10 February 2022** | **23 February 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/102023**

**C.    DOCUMENTS CONSIDERED TO BE RELEVANT**

| Category* | Citation of document, with indication, where appropriate, of the relevant passages | Relevant to claim No. |
|---|---|---|
| Y | CN 109563043 A (GLAXOSMITHKLINE IP DEV LTD. et al.) 02 April 2019 (2019-04-02) embodiment 3, and claims 1-6 and 40 | 7-12 |
| Y | CN 109715173 A (VIRACTA THERAPEUTICS INC.) 03 May 2019 (2019-05-03) claim 1 | 7-12 |
| A | 林志烽 等, (LIN, Zhifeng et al.,). "DNA甲基转移酶抑制剂抗肿瘤的免疫机制及其在肿瘤免疫治疗中的应用, (Anti-Tumor Immune Mechanism of DNA Methyltransferase Inhibitors and Their Application in Tumor Immunotherapy,)" 细胞与分子免疫学杂志 *(Chinese Journal of Cellular and Molecular Immunology),* Vol. 33, No. 12, 31 December 2017 (2017-12-31), pp. 1706-1710 | 1-13 |

Form PCT/ISA/210 (second sheet) (January 2015)

<table>
<tr><td colspan="3" align="center"><b>INTERNATIONAL SEARCH REPORT</b><br>Information on patent family members</td><td colspan="2">International application No.<br><b>PCT/CN2021/102023</b></td></tr>
</table>

| Patent document cited in search report | | | Publication date (day/month/year) | Patent family member(s) | | | Publication date (day/month/year) |
|---|---|---|---|---|---|---|---|
| CN | 109563043 | A | 02 April 2019 | CO | 2018013717 | A2 | 18 January 2019 |
| | | | | CL | 2018003577 | A1 | 05 April 2019 |
| | | | | EP | 3468953 | A1 | 17 April 2019 |
| | | | | AU | 2017283790 | A1 | 22 November 2018 |
| | | | | AU | 2017283790 | B2 | 29 August 2019 |
| | | | | KR | 20190017030 | A | 19 February 2019 |
| | | | | MX | 2018015483 | A | 18 March 2019 |
| | | | | JO | P20180120 | A1 | 30 January 2019 |
| | | | | CR | 20180580 | A | 02 July 2019 |
| | | | | WO | 2017216727 | A1 | 21 December 2017 |
| | | | | WO | 2017216726 | A1 | 21 December 2017 |
| | | | | JP | 2019517596 | A | 24 June 2019 |
| | | | | CA | 3026226 | A1 | 21 December 2017 |
| | | | | PE | 20190971 | A1 | 09 July 2019 |
| | | | | DO | P2018000273 | A | 31 March 2019 |
| | | | | MA | 45244 | A | 17 April 2019 |
| | | | | CA | 3026211 | A1 | 21 December 2017 |
| | | | | BR | 112018075992 | A2 | 02 April 2019 |
| | | | | SG | 11201809559 U | A | 28 December 2018 |
| | | | | PH | 12018502633 | A1 | 30 September 2019 |
| | | | | US | 2019194166 | A1 | 27 June 2019 |
| | | | | US | 10975056 | B2 | 13 April 2021 |
| | | | | IL | 263163 | D0 | 31 December 2018 |
| | | | | IL | 263163 | A | 31 October 2021 |
| CN | 109715173 | A | 03 May 2019 | JP | 2019522054 | A | 08 August 2019 |
| | | | | JP | 2019525957 | A | 12 September 2019 |
| | | | | WO | 2018013962 | A1 | 18 January 2018 |
| | | | | GB | 201901327 | D0 | 20 March 2019 |
| | | | | GB | 2567362 | A | 10 April 2019 |
| | | | | US | 2019290646 | A1 | 26 September 2019 |
| | | | | WO | 2018013975 | A1 | 18 January 2018 |
| | | | | EP | 3484489 | A1 | 22 May 2019 |
| | | | | EP | 3484489 | A4 | 01 April 2020 |
| | | | | GB | 201901338 | D0 | 20 March 2019 |
| | | | | GB | 2567093 | A | 03 April 2019 |
| | | | | CN | 109803661 | A | 24 May 2019 |
| | | | | EP | 3484478 | A1 | 22 May 2019 |
| | | | | EP | 3484478 | A4 | 01 April 2020 |
| | | | | US | 2019216818 | A1 | 18 July 2019 |
| WO | 2020165402 | A1 | 20 August 2020 | None | | | |

Form PCT/ISA/210 (patent family annex) (January 2015)